# EUROPÄISCHE PATENTANMELDUNG

(11) **EP 2 111 059 A2**
(43) Veröffentlichungstag der Anmeldung: **21.10.2009**
(21) Anmeldenummer: 09155623.3
(22) Anmeldetag: 19.03.2009
(51) Int. Cl.: H04R 25/00

(54) **Verfahren zur Bestimmung einer Zeitkonstante des Gehörs und Verfahren zum Einstellen einer Hörvorrichtung**

(30) Priorität: 17.04.2008 DE 102008019374
(71) Anmelder: Siemens Medical Instruments Pte. Ltd., Singapore 139959 (SG)
(72) Erfinder: Latzel, Matthias, 91330, Eggolsheim (DE)
(74) Vertreter: Maier, Daniel Oliver

(57) **Zusammenfassung**

Das Zeitauflösungsvermögen bei Hörgeräteträgern soll optimiert werden. Hierzu ist ein Verfahren zum Einstellen einer Signalverarbeitungseinrichtung einer Hörvorrichtung und insbesondere eines Hörgeräts mit folgenden Schritten vorgesehen: Darbieten eines akustischen Signals (S2), Verarbeiten des akustischen Signals mit der Signalverarbeitungseinrichtung, die eine erste Zeitkonstante (τᵢ) besitzt zu einem verarbeiteten ersten Schallsignal (S3) und Zuordnen des ersten Schallsignals zu einer ersten Lautheit durch den Nutzer (S4). Anschließend erfolgt ein erneutes Darbieten des akustischen Signals (S2), ein Verarbeiten des akustischen Signals mit der Signalverarbeitungseinrichtung, deren Zeitkonstante gegenüber der ersten Zeitkonstante (τᵢ) auf eine kürzere zweite Zeitkonstante (τᵢ₊₁) reduziert ist, zu einem verarbeiteten zweiten Schallsignal (S5) sowie ein Zuordnen des zweiten Schallsignals zu einer zweiten Lautheit durch den Nutzer (S6). Diese Schritte werden so lange wiederholt, bis die zweite Lautheit erstmals gleich der ersten Lautheit ist (S7), wobei bei jeder Wiederholung die erste Zeitkonstante verkürzt wird (S8). Schließlich wird die Signalsverarbeitungseinrichtung mit der resultierenden ersten oder zweiten Zeitkonstante eingestellt (S9). Auf diese Weise kann nicht nur die optimale Einstellung der Zeitkonstante eines Hörgeräts, sondern auch die Zeitkonstante des Gehörs eines Nutzers auf der Basis der Lautheit bestimmt werden.

## Beschreibung

Die vorliegende Erfindung betrifft ein Verfahren zur Bestimmung einer Zeitkonstante des Gehörs eines Nutzers. Darüber hinaus betrifft die vorliegende Erfindung ein Verfahren zum Einstellen eines Signalverarbeitungsalogrithmus einer Hörvorrichtung. Unter dem Begriff "Hörvorrichtung" wird hier jedes im oder am Ohr tragbare Gerät, insbesondere ein Hörgerät, ein Headset, Kopfhörer und dergleichen, verstanden.

Hörgeräte sind tragbare Hörvorrichtungen, die zur Versorgung von Schwerhörenden dienen. Um den zahlreichen individuellen Bedürfnissen entgegenzukommen, werden unterschiedliche Bauformen von Hörgeräten wie Hinter-dem-Ohr-Hörgeräte (HdO), Hörgerät mit externem Hörer (RIC: receiver in the canal) und In-dem-Ohr-Hörgeräte (IdO), z.B. auch Concha-Hörgeräte oder Kanal-Hörgeräte (ITE, CIC), bereitgestellt. Die beispielhaft aufgeführten Hörgeräte werden am Außenohr oder im Gehörgang getragen. Darüber hinaus stehen auf dem Markt aber auch Knochenleitungshörhilfen, implantierbare oder vibrotaktile Hörhilfen zur Verfügung. Dabei erfolgt die Stimulation des geschädigten Gehörs entweder mechanisch oder elektrisch.

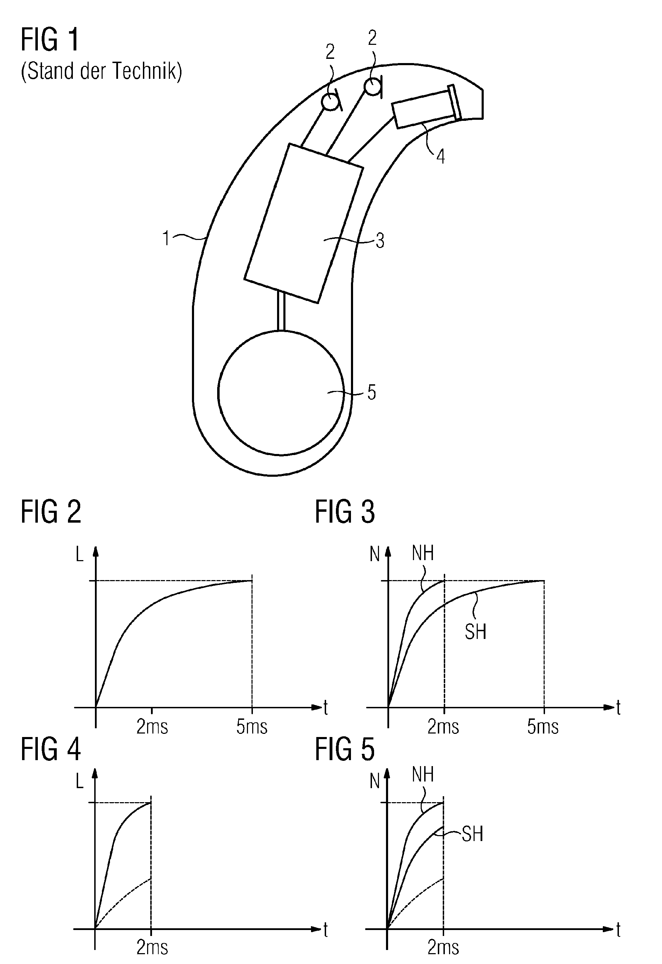

Hörgeräte besitzen prinzipiell als wesentliche Komponenten einen Eingangswandler, einen Verstärker und einen Ausgangswandler. Der Eingangswandler ist in der Regel ein Schallempfänger, z. B. ein Mikrofon, und/oder ein elektromagnetischer Empfänger, z. B. eine Induktionsspule. Der Ausgangswandler ist meist als elektroakustischer Wandler, z. B. Miniaturlautsprecher, oder als elektromechanischer Wandler, z. B. Knochenleitungshörer, realisiert. Der Verstärker ist üblicherweise in eine Signalverarbeitungseinheit integriert. Dieser prinzipielle Aufbau ist in FIG 1 am Beispiel eines Hinter-dem-Ohr-Hörgeräts dargestellt. In ein Hörgerätegehäuse 1 zum Tragen hinter dem Ohr sind ein oder mehrere Mikrofone 2 zur Aufnahme des Schalls aus der Umgebung eingebaut. Eine Signalverarbeitungseinheit 3, die ebenfalls in das Hörgerätegehäuse 1 integriert ist, verarbeitet die Mikrofonsignale und verstärkt sie. Das Ausgangssignal der Signalverarbeitungseinheit 3 wird an einen Lautsprecher bzw. Hörer 4 übertragen, der ein akustisches Signal ausgibt. Der Schall wird gegebenenfalls über einen Schallschlauch, der mit einer Otoplastik im Gehörgang fixiert ist, zum Trommelfell des Geräteträgers übertragen. Die Energieversorgung des Hörgeräts und insbesondere die der Signalverarbeitungseinheit 3 erfolgt durch eine ebenfalls ins Hörgerätegehäuse 1 integrierte Batterie 5.

Die wirksamen Zeitkonstanten von Signalverarbeitungseinrichtungen und insbesondere von Kompressionsschaltungen in Hörgeräten werden derzeit rein technisch bestimmt (EN 60118-2:1995). Dafür gibt es eine Reihe von Normen, in denen die Messvorschriften genau definiert sind. Bei den rein technischen Messungen wird demnach ein Wert bestimmt, der nur einen theoretischen Bezug hat, jedoch keine Aussage über die Wirksamkeit beim individuellen Schwerhörigen liefert. Da diese Messungen nicht perzeptiv sind, kann durch sie nicht bestimmt werden, welche Zeitkonstante vom Schwerhörigen noch verarbeitet werden kann. Die einzige Methode derzeit, die für die Evaluation der Wirksamkeit von Zeitkonstanten von Kompressionsschaltungen herangezogen wird, ist die Verwendung von Fragebögen. In diesen kann jedoch nur indirekt nach der Effektivität von Zeitkonstanten gefragt werden. Zudem ist diese Methode durch die bekannte Ungenauigkeit von Fragebögen belastet.

Bekanntermaßen lässt sich die kategoriale Lautheitsskalierung KLS, die eine Zuordnung einer Wahrnehmungsgröße (z. B. laut, mittel, leise) zu einer physikalischen Größe (z. B. Schalldruckpegel) darstellt, dazu verwenden, das Lautheitsempfinden des pathologischen Gehörs auszumessen oder die Wirksamkeit von Hörgeräten zu bestimmen (ISO 16832 2006). Dabei werden einerseits geeignete Schmalbandsignale gewählt, um frequenzspezifische Daten zu erhalten, und andererseits ausreichend lange Signale gewählt, um die Wirksamkeit des Hörgeräts im eingeschwungenen Zustand zu bestimmen. Beispielsweise lässt sich mit ausreichend langen Signalen die Reaktion von Störgeräuschunterdrückungsalgorithmen abwarten.

Die Aufgabe der vorliegenden Erfindung besteht somit darin, ein Verfahren anzugeben, mit dem die Zeitkonstante des Gehörs eines Nutzers mit erhöhter Genauigkeit bestimmt werden kann. Darüber hinaus soll ein Verfahren angegeben werden, mit dem die Signalverarbeitungseinrichtung einer Hörvorrichtung im Hinblick auf einen reduzierten Energieverbrauch besser eingestellt werden kann.

Erfindungsgemäß wird diese Aufgabe gelöst durch ein Verfahren zur Bestimmung einer Zeitkonstante des Gehörs eines Nutzers mit den Schritten: Darbieten eines akustischen Signals, Verarbeiten des akustischen Signals mit einer Signalverarbeitungseinrichtung, die eine erste Zeitkonstante besitzt, zu einem verarbeiteten ersten Schallsignal, Zuordnen des ersten Schallsignals zu einer ersten Lautheit durch den Nutzer, erneutes Darbieten des akustischen Signals, Verarbeiten des akustischen Signals mit der Signalverarbeitungseinrichtung, deren Zeitkonstante gegenüber der ersten Zeitkonstante auf eine kürzere zweite Zeitkonstante reduziert ist, zu einem verarbeiteten zweiten Schallsignal, Zuordnen des zweiten Schallsignals zu einer zweiten Lautheit durch den Nutzer, Wiederholen der vorhergehenden Schritte solange bis die zweite Lautheit erstmals gleich der ersten Lautheit ist, wobei bei jeder Wiederholung die erste Zeitkonstante verkürzt wird, und Festlegen der resultierenden ersten oder zweiten Zeitkonstante als die Zeitkonstante des Gehörs des Nutzers.

In vorteilhafter Weise ist es so möglich, die Zeitauflösung des Gehörs anhand der Lautheitsempfindung des individuellen Gehörs zu bestimmen.

Darüber hinaus wird erfindungsgemäß bereitgestellt ein Verfahren zum Einstellen einer Signalverarbeitungseinrichtung einer Hörvorrichtung mit den Schritten: Darbieten eines akustischen Signals, Verarbeiten des akustischen Signals mit der Signalverarbeitungseinrichtung, die eine erste Zeitkonstante besitzt, zu einem verarbeiteten ersten Schallsignal, Zuordnen des ersten Schallsignals zu einer ersten Lautheit durch den Nutzer, erneutes Darbieten des akustischen Signals, Verarbeiten des akustischen Signals mit der Signalverarbeitungseinrichtung, deren Zeitkonstante gegenüber der ersten Zeitkonstante auf eine kürzere zweite Zeitkonstante reduziert ist, zu einem verarbeiteten zweiten Schallsignal, Zuordnen des zweiten Schallsignals zu einer zweiten Lautheit durch den Nutzer, Wiederholen der vorhergehenden Schritte solange bis die zweite Lautheit erstmals gleich der ersten Lautheit ist, wobei bei jeder Wiederholung die erste Zeitkonstante verkürzt wird, und Einstellen der Signalverarbeitungseinrichtung mit der resultierenden ersten oder zweiten Zeitkonstante.

Hierdurch ist es möglich, eine Hörvorrichtung und insbesondere ein Hörgerät exakt auf das temporale Hörvermögen eines Nutzers einzustellen. Insbesondere kann hierdurch vermieden werden, dass die Hörvorrichtung eine geringere Zeitauflösung besitzt als der Nutzer überhaupt wahrnehmen kann. Auf diese Weise lässt sich zusätzlich die Operationsrate und damit auch der Energieverbrauch ohne Qualitätsverluste senken.

Vorzugsweise sind die erste und die zweite Lautheit bei den oben beschriebenen Verfahren Elemente einer kategorialen Lautheitsskalierung. Dies bedeutet, dass der Nutzer sich beispielsweise lediglich zwischen den Werten "laut", "mittel" und "leise" bei seiner Bewertung der Lautheit entscheiden muss.

Die Signaldauer des akustischen Signals ist günstigerweise kleiner oder gleich 5 ms bzw. 15 ms. Bei derart kurzen Zeitdauern wirken sich die Zeitkonstanten der Systeme deutlich auf die Lautheit aus. In der Praxis kann natürlich eine Folge derartiger Impulse mit gleichen Schalldruckpegeln dargeboten werden, um dem Nutzer die Zuordnung zu einer entsprechenden Lautheit zu erleichtern.

Das Verarbeiten des akustischen Signals kann eine automatische Verstärkungssteuerung umfassen. Gerade derartige Verstärkungssteuerungen (AGC; Automatic Game Control) weisen für die jeweilige Hörvorrichtung maßgebliche Zeitkonstanten auf, die es soweit wie möglich zu optimieren gilt. Eine solche Zeitkonstante ist meist auch durch eine etwaige Dynamikkompression bestimmt.

Die oben genannten Verfahren können auch für mehrere Pegel des akustischen Signals durchgeführt werden. Damit können Pegellautheitskurven aufgenommen und für eine Optimierung herangezogen werden. Die gesuchte Zeitkonstante ist dann unter Umständen ein Kompromiss zwischen bzw. ein Mittelwert von mehreren Zeitkonstanten. In der vorliegenden Anmeldung soll jedoch auch unter dem Begriff "Lautheit" eine Lautheitsfunktion über dem Pegel verstanden werden. In diesem Fall lässt sich dann die gesuchte Zeitkonstante dadurch finden, dass die Zeitkonstante der Hörvorrichtung so lange reduziert wird, bis sich die Pegellautheitsfunktion zumindest bereichsweise nicht mehr verändert.

Das akustische Signal kann auch mehrere Einzelsignale unterschiedlichen Pegels umfassen, wobei die zugeordneten Lautheiten Pegellautheitsfunktionen sind. Damit lässt sich die gesuchte Zeitkonstante anhand mindestens eines Teilbereichs von Pegellautheitsfunktionen bestimmen bzw. einstellen.

Die oben genannten Verfahren zum Bestimmen oder Einstellen von Zeitkonstanten können alternativ auch dadurch realisiert werden, dass die Zeitkonstanten schrittweise erhöht werden. Konkret ist dann die zweite Zeitkonstante nicht kürzer sondern länger als die erste Zeitkonstante, und das Wiederholen findet so oft statt, nicht bis beide Lautheiten gleich sind, sondern bis sich die zweite Lautheit von der ersten Lautheit erstmals unterscheidet, wobei bei jeder Wiederholung die erste Zeitkonstante eben nicht verkürzt sondern verlängert wird.

Die vorliegende Erfindung wird anhand der beigefügten Zeichnungen näher erläutert, in denen zeigen:
- FIG 1: den prinzipiellen Aufbau eines Hörgeräts gemäß dem Stand der Technik;
- FIG 2: ein Zeitdiagramm des Lautstärkepegels eines Schallim- pulses;
- FIG 3: ein Zeitdiagramm der Lautheitsempfindungen eines Nor- mal- und eines Schwerhörenden;
- FIG 4: ein Zeitdiagramm des Lautstärkepegels eines gegenüber dem von FIG 2 verkürzten Schallimpulses, der mit ver- kürzter Zeitkonstante erzeugt ist;
- FIG 5: ein Zeitdiagramm der Lautheitsempfindungen eines Nor- mal- und eines Schwerhörenden bezüglich des Schallim- pulses von FIG 4;
- FIG 6: ein Ablaufdiagramm zur Bestimmung einer Zeitkonstante bzw. zum Einstellen einer Hörvorrichtung und
- FIG 7: ein Pegellautheitsdiagramm zur Darstellung des Ein- flusses der Zeitkonstante einer Signalverarbeitung.

Die nachfolgend näher geschilderten Ausführungsbeispiele stellen bevorzugte Ausführungsformen der vorliegenden Erfindung dar.

Es ist bekannt, dass im pathologischen Gehör das Zeitauflösungsvermögen beeinträchtigt ist. Durch eine geeignete Signalverarbeitung eines Hörgeräts kann ein Verlust an Zeitauflösungsvermögen (teilweise) kompensiert werden. Dazu kann es notwendig sein, die wirksame Zeitkonstante des Gehörs eines Nutzers bzw. Hörgeräteträgers zu bestimmen bzw. die Zeitkonstante der Signalverarbeitung des Hörgeräts in geeigneter Weise einzustellen.

Anhand der FIG 2 bis 5 wird nun schematisch die Bedeutung der Zeitkonstanten des Gehörs und der Signalverarbeitung in einem Hörgerät insbesondere der AGC erläutert. Es wird beispielsweise gemäß FIG 2 ein Testschallimpuls erzeugt, der aufgrund der verarbeitungs- bzw. gerätespezifischen Zeitkonstante seinen Sollschalldruckpegel nach ca. 5 ms erreicht.

In FIG 3 ist eine Kurve NH dargestellt, die die Zeitkonstante eines Gehörs eines Normalhörenden verdeutlicht. Ca. 2 ms nach einem sprunghaften Anstieg des physikalischen Schalldruckpegels ändert sich die empfundene Lautheit N praktisch nicht mehr. Die Kurve SH zeigt demgegenüber, dass das Integrationsvermögen des Gehörs eines Schwerhörenden reduziert ist, und beispielsweise erst nach 5 ms ein Sättigungswert erreicht wird. Demnach empfindet der Schwerhörende 2 ms nach Beginn des Pegelsprungs noch nicht die gleiche Lautheit wie der Normalhörende. Nach 5 ms empfinden jedoch beide die gleiche Lautheit.

FIG 4 gibt nun einen Testschall wieder, der durch ein Gerät bzw. eine Verarbeitungseinheit mit gegenüber FIG 2 verringerter Zeitkonstante erzeugt wurde. Der Sollschalldruckpegel ist hier bereits nach 2 ms erreicht.

Aus FIG 5 ist zu entnehmen, dass der Normalhörende (Kurve NH) den kurzen Testschall von FIG 4 deutlich lauter wahrnimmt als der Schwerhörende (Kurve SH). Dies bedeutet, dass mit reduziertem Integrationsvermögen des Gehörs der gleiche Testschall leiser empfunden wird. Der Schwerhörige könnte also von einer geringeren Zeitkonstante der Signalverarbeitung nur profitieren, solange diese länger ist als die Zeitkonstante seines Gehörs.

Erfindungsgemäß soll nun die Zeitkonstante der Signalverarbeitung anhand des Lautheitsempfindens an das Zeitauflösungsvermögen des Gehörs des Hörgeräteträgers angepasst werden. Dies kann dadurch erfolgen, dass die maximale Steilheit der Kurve SH anhand einer Variation der Steilheit der Kurven der FIG 2 und 4 ermittelt wird.

In einem Ausführungsbeispiel wird nun die KLS für die Überprüfung der Wirksamkeit von Zeitkonstanten oder allgemein zur Bestimmung der Zeitverarbeitung des pathologischen Gehörs herangezogen. Dem Hörgeräteträger werden spezielle, kurze Signale (mit Transienten) dargeboten, z. B. eine Folge von Trommelschlägen. Diese sehr kurzen Signale regen die Kompression im Hörgerät je nach eingestellter Zeitkonstante entsprechend an. Die Testperson, also der Hörgeräteträger, empfindet in Abhängigkeit von der eingestellten Zeitkonstante eine gewisse Lautheit der KLS. Aus dem Ergebnis, d. h. der empfundenen Lautheit der Testperson, kann man schließlich ablesen, ob die Zeitkonstante (subjektiv) richtig eingestellt ist.

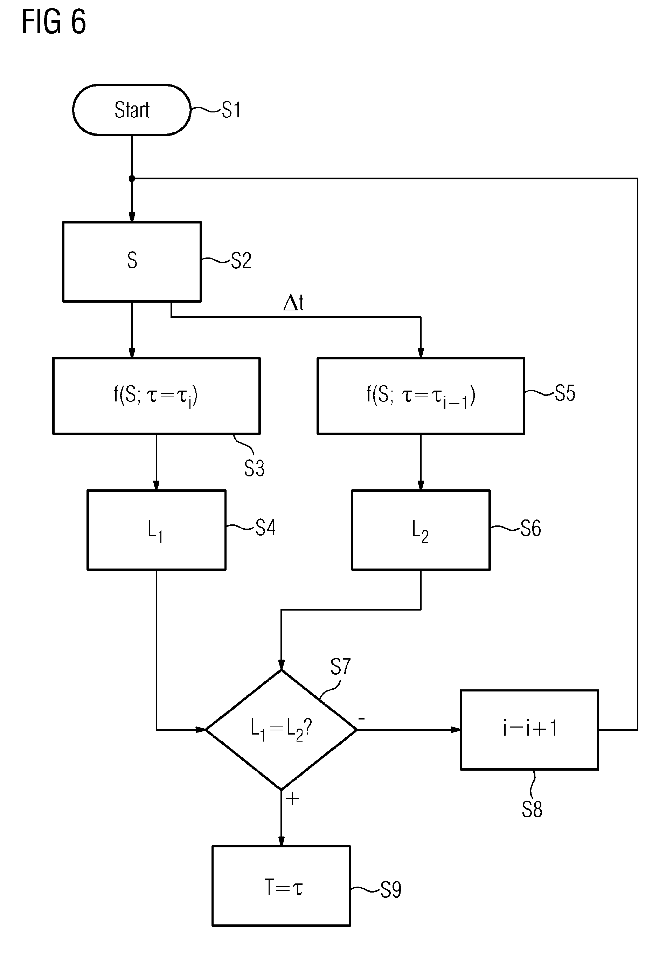

In FIG 6 ist ein Ablaufdiagramm zu dem erfindungsgemäßen Verfahren dargestellt. Nach dem Start S1 des Verfahrens wird der Testperson ein Testschall s in Schritt S2 dargeboten. Das Hörgerät verarbeitet das kurze Schallsignal s mit seiner Signalverarbeitungseinrichtung, die eine Zeitkonstante τᵢ besitzt (vgl. Schritt S3). Die Testperson empfindet das verarbeitete Schallsignal entsprechend der kategorialen Lautheit L₁ (Schritt S4). Nun wird die Zeitkonstante der Signalverarbeitungseinrichtung des Hörgeräts verkürzt. Sie beträgt nun τᵢ₊₁. Daraufhin wird der Testperson das Testsignal s erneut präsentiert, was in FIG 6 durch ein Δt nach Schritt S2 angedeutet ist. Gemäß Schritt S5 findet nun die Signalverarbeitung mit der verkürzten Zeitkonstante τᵢ₊₁ statt. Das resultierende Schallsignal empfindet die Testperson entsprechend der Lautheit L₂ (Schritt S6).

In Schritt S7 wird verglichen, ob die beiden Lautheiten L₁ und L₂ gleich sind. Ist dies nicht der Fall, so verläuft die Lautheit entsprechend dem vereinfachten Beispiel von FIG 5 gemäß der gestrichelten Kurve. Deshalb werden die Schritte S2 bis S7 mit jeweils verkürzten Zeitkonstanten wiederholt, was in FIG 6 mit dem Iterationsschritt i=i+1 (Schritt S8) angedeutet ist. Dies bedeutet, dass bei dem erneuten Durchlauf sowohl in Schritt S3 als auch in Schritt S5 jeweils die Zeitkonstante im Verhältnis zum vorhergehenden Durchlauf verkürzt ist.

Empfindet die Testperson schließlich die beiden dargebotenen Schalle als gleich laut, d. h. L₁=L₂, so entspricht die Zeitkonstante der Signalverarbeitungseinrichtung derjenigen des Gehörs des Schwerhörigen oder liegt geringfügig darüber (innerhalb der Schrittweite). Die Zeitkonstante braucht nicht weiter verkürzt zu werden. Weitere Rechenoperationen, die für eine weitere Verkürzung der Zeitkonstante notwendig wären, müssen in dem Hörgerät also nicht durchgeführt werden, wodurch Verarbeitungszeit und Energie eingespart werden kann.

Durch die Variation der Zeitkonstante der Kompression bzw. der Signalverarbeitung kann auch das minimale Zeitauflösungsvermögen des Schwerhörigen bestimmt werden. Dazu wird wie soeben für das Einstellen des Hörgeräts beschrieben die Zeitkonstante so lange verringert, bis sich die Lautheit nicht mehr verändert. In diesem Fall ist dann die Verarbeitungszeit des Hörgeräts zu kurz und kann vom pathologischen Gehör nicht mehr aufgelöst werden. Die ermittelte Zeitkonstante τ kann dann als Zeitkonstante des Gehörs festgesetzt werden (Schritt S9).

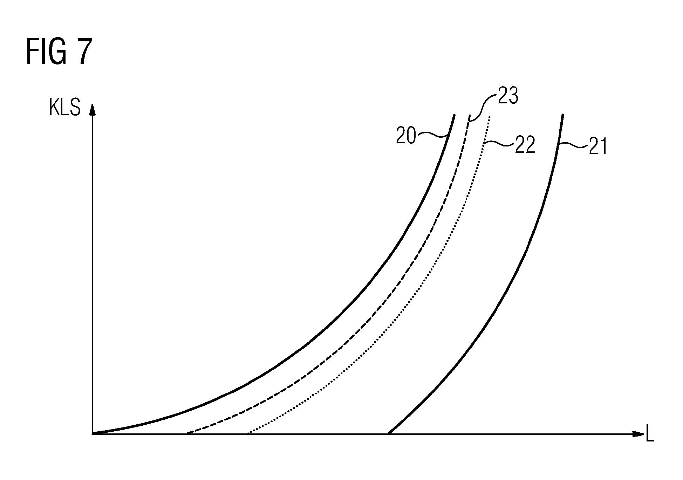

Eine weitere Ausführungsform der vorliegenden Erfindung ist in FIG 7 angedeutet. Dort ist eine Pegellautheitsfunktion, hier eine KLS-Zielfunktion 20 eines Normalhörenden über dem Pegel L für ein bestimmtes Signal dargestellt. Des Weiteren ist die pathologische Pegellautheitsfunktion 21 des Schwerhörigen für dasselbe Signal eingezeichnet. Mit einem Hörgerät geringer Zeitauflösung lässt sich nun eine Pegellautheitsfunktion 22 erzielen. Verkürzt man die Zeitkonstante des Hörgeräts, so wird die Lautheitswahrnehmung so verändert, dass sich eine Pegellautheitsfunktion entsprechend der Kurve 23 ergibt. Das Minimum der Zeitauflösung ist dann erreicht, wenn die Lage und Steilheit der Pegellautheitsfunktion durch eine Verringerung der Zeitkonstante nicht mehr verändert werden kann.

Um die gesuchten Zeitkonstanten zu finden, kann als Ausgangspunkt auch eine Signalverarbeitungseinrichtung mit sehr kurzer Zeitkonstante verwendet werden. Die Zeitkonstante wird dann sukzessive vergrößert. Solang die Zeitauflösung der Signalverarbeitung wesentlich kürzer ist als diejenige des Gehörs des Hörgeräteträgers, wird der Hörgeräteträger keinen Lautheitsunterschied wahrnehmen. Erst wenn die Zeitauflösung der Signalverarbeitung geringer wird als diejenige des Gehörs, wird sich eine Reduktion der Lautheit einstellen. Diese Schwelle ist dann wiederum durch die gesuchte Zeitkonstante gekennzeichnet.

Durch die oben geschilderten erfindungsgemäßen Verfahren kann einerseits die minimale Zeitkonstante in Kompressionsschaltungen bestimmt werden, die von einem individuellen, pathologischen Gehör noch aufgelöst werden kann. Andererseits können diese Verfahren auch dafür verwendet werden, um die Zeitkonstante an die individuellen Bedürfnisse des Hörgeräteträgers anzupassen, indem eine KLS als Zielfunktion (Normalhörenden-KLS) vorgegeben und die Zeitkonstante in der Kompressionsschaltung so lange (interaktiv) variiert wird, bis die Zielfunktion vom Schwerhörigen mit Hörgerät erreicht wird.

## Patentansprüche

1. Verfahren zur Bestimmung einer Zeitkonstante des Gehörs eines Nutzers mit den Schritten:
- Darbieten (S2) eines akustischen Signals,
- Verarbeiten (S3) des akustischen Signals mit einer Signalverarbeitungseinrichtung, die eine erste Zeitkonstante besitzt, zu einem verarbeiteten ersten Schallsignal,
- Zuordnen (S4) des ersten Schallsignals zu einer ersten Lautheit durch den Nutzer,
- erneutes Darbieten (S2) des akustischen Signals,
- Verarbeiten (S5) des akustischen Signals mit der Signalverarbeitungseinrichtung, deren Zeitkonstante gegenüber der ersten Zeitkonstante auf eine kürzere zweite Zeitkonstante reduziert ist, zu einem verarbeiteten zweiten Schallsignal,
- Zuordnen (S6) des zweiten Schallsignals zu einer zweiten Lautheit durch den Nutzer,
- Wiederholen (S8) der vorhergehenden Schritte solange bis die zweite Lautheit erstmals gleich der ersten Lautheit ist (S7), wobei bei jeder Wiederholung die erste Zeitkonstante verkürzt wird, und
- Festlegen (S9) der resultierenden ersten oder zweiten Zeitkonstante als die Zeitkonstante des Gehörs des Nutzers.

2. Verfahren zum Einstellen einer Signalverarbeitungseinrichtung einer Hörvorrichtung mit den Schritten:
- Darbieten (S2) eines akustischen Signals,
- Verarbeiten (S3) des akustischen Signals mit der Signalverarbeitungseinrichtung, die eine erste Zeitkonstante besitzt, zu einem verarbeiteten ersten Schallsignal,
- Zuordnen (S4) des ersten Schallsignals zu einer ersten Lautheit durch den Nutzer,
- erneutes Darbieten (S2) des akustischen Signals,
- Verarbeiten (S5) des akustischen Signals mit der Signalverarbeitungseinrichtung, deren Zeitkonstante gegenüber der ersten Zeitkonstante auf eine kürzere zweite Zeitkonstante reduziert ist, zu einem verarbeiteten zweiten Schallsignal,
- Zuordnen (S6) des zweiten Schallsignals zu einer zweiten Lautheit durch den Nutzer,
- Wiederholen (S8) der vorhergehenden Schritte solange bis die zweite Lautheit erstmals gleich der ersten Lautheit ist (S7), wobei bei jeder Wiederholung die erste Zeitkonstante verkürzt wird, und
- Einstellen der Signalverarbeitungseinrichtung mit der resultierenden ersten oder zweiten Zeitkonstante.

3. Verfahren nach Anspruch 1 oder 2, wobei die erste und die zweite Lautheit Elemente einer kategorialen Lautheitsskalierung sind.

4. Verfahren nach einem der Ansprüche 1 bis 3, wobei die Signaldauer des akustischen Signals kleiner oder gleich 15 ms ist.

5. Verfahren nach einem der vorhergehenden Ansprüche, wobei das Verarbeiten des akustischen Signals eine automatische Verstärkungssteuerung umfasst.

6. Verfahren nach einem der vorhergehenden Ansprüche, wobei das Verarbeiten eine Dynamikkompression umfasst.

7. Verfahren nach einem der vorhergehenden Ansprüche, das für mehrere Pegel des akustischen Signals durchgeführt wird.

8. Verfahren nach einem der vorhergehenden Ansprüche, wobei das akustische Signal mehrere Einzelsignale unterschiedlichen Pegels umfasst und die zugeordneten Lautheiten Pegellautheitsfunktionen sind.

9. Verfahren nach einem der vorhergehenden Ansprüche, wobei die zweite Zeitkonstante nicht kürzer sondern länger ist als die erste Zeitkonstante, und das Wiederholen so oft stattfindet, nicht bis beide Lautheiten gleich sind, sondern bis sich die zweite Lautheit von der ersten Lautheit erstmals unterscheidet, wobei bei der Wiederholung die erste Zeitkonstante nicht verkürzt, sondern verlängert wird.
